(19) Europäisches Patentamt
European Patent Office
Office européen des brevets

(11) Publication number: **0 173 937**
**A2**

(12) # EUROPEAN PATENT APPLICATION

(21) Application number: 85110684.9

(22) Date of filing: 26.08.85

(51) Int. Cl.[4]: C 12 N 1/38
C 12 N 1/18
//C12N1/06, C12P21/02

(30) Priority: 07.09.84 US 648754

(43) Date of publication of application: 12.03.86 Bulletin 86/11

(84) Designated Contracting States: AT CH DE FR GB IT LI NL SE

(71) Applicant: MILES LABORATORIES, INC.
1127 Myrtle Street
Elkhart Indiana 46514(US)

(72) Inventor: Boguslawski, George
2323 Kenilworth Drive
Elkhart Indiana 46514(US)

(74) Representative: Dänner, Klaus, Dr. et al,
c/o Bayer AG Konzernverwaltung RP Patentabteilung
D-5090 Leverkusen 1 Bayerwerk(DE)

(54) **Method of increasing the permeability of yeast cells.**

(57) A method for increasing the permeability of yeast cells by incubating the yeast cells with polymyxin B nonapeptide is disclosed. Also taught is a method for increasing the efficiency of secretion from yeast cells of polypeptides encoded for by genomic DNA or cloned genes present in said cells.

Croydon Printing Company Ltd.

0173937

# METHOD OF INCREASING THE PERMEABILITY OF YEAST CELLS

## BACKGROUND OF THE INVENTION

The expression of chromosomal genes of higher eucaryotes is often conveniently studied in a eucaryotic host which is relatively easy to manipulate such as yeast. Utilizing yeast as a cloning host offers certain advantages such as a secretory pathway which follows the same general route as that involved in animal cells. Therefore, where post-translational modification of a eucaryotic protein is required for biological activity (as in interferon), yeast is a more appropriate host than bacteria. When relatively large quantities of scarce protein are to be produced, enhanced secretion of that protein from the host is desirable. However, to this point producing such large quantities in yeast has been problematical.

The present invention discloses a method of increasing the permeability of yeast cells due to the effect thereon of polymyxin B nonapeptide (hereafter alternatively referred to as "PBN"). By increasing the permeability of yeast cells,

secretion of polypeptides from yeast encoded by genomic DNA or cloned genes is enhanced thus facilitating recovery and purification of the desired polypeptide products.

Polymyxin B is a complex antibiotic substance elaborated by various strains of *Bacillus polymyxa* and is toxic to bacterial, fungal and animal cells. This toxicity is attributable to the ability of the compound to disrupt the integrity of cell membranes thereby allowing cell contents to escape. The antibiotic consists of a cyclic peptide portion and a fatty acyl portion joined by an amide bond. This amide bond can be cleaved by papain or ficin resulting in a cyclic nonapeptide referred to as polymyxin B nonapeptide or simply PBN. PBN has been shown to be nontoxic to bacteria but sensitizes the bacterial cells to other antibiotics or serum complement. (Vaara and Vaara, Nature, Vol. 303, 526-528, 1983).

## SUMMARY OF THE INVENTION

The present invention is directed to a method of increasing the permeability of yeast cells by incubating the yeast cells with an effective amount of polymyxin B nonapeptide. The invention also discloses a method for increasing the efficiency of secretion from yeast of polypeptides encoded by genomic DNA or cloned genes by incubating said yeast containing said genomic DNA or cloned genes in the presence of an effective amount of polymyxin B nonapeptide and recovering therefrom said polypeptide.

## DETAILED DESCRIPTION OF THE INVENTION

Polymyxin B nonapeptide is prepared from the antibiotic polymyxin B by the enzymatic cleavage of the amide bond joining the fatty acyl moiety and the peptide moiety of the molecule. The reaction may be illustrated as follows (where "dab" refers to diaminobutyric acid and "FA" refers to fatty acid):

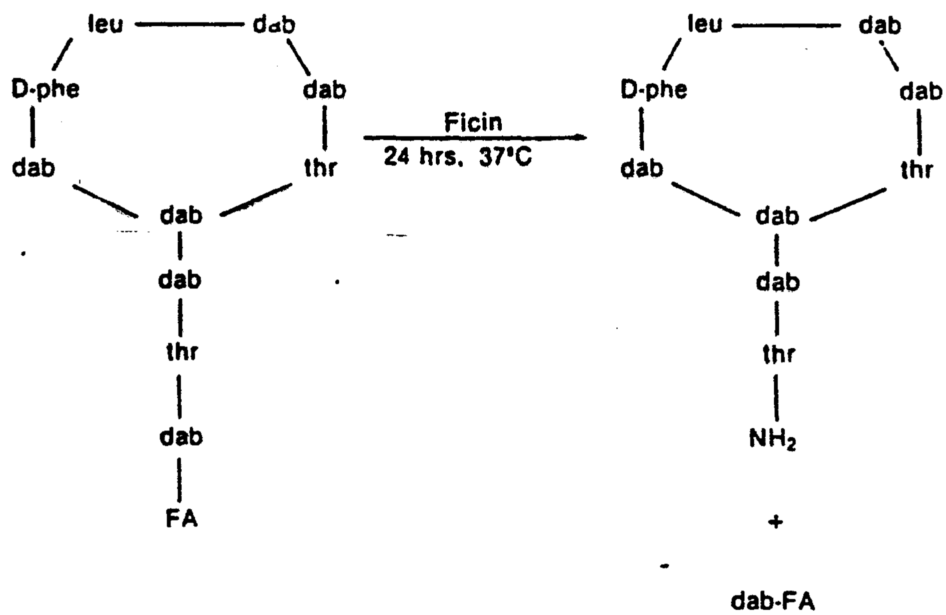


The preparation of PBN is accomplished by the method of Chihara, et al, Agr. Biol. Chem., 37 (11), 2455-2463 (1973), which is incorporated herein by reference.

The present invention provides a method for increasing the permeability of yeast cells thereby affecting an increase in the efficiency of secretion of polypeptides encoded by either genomic DNA

or cloned genes within the yeast cell. For these purposes, the yeast cell is incubated with an effective amount of PBN sufficient to increase the secretion of said polypeptide from the cell without the PBN itself being toxic to the cell. As used herein, the phrase "effective amount" refers to from about 0.05 to about 0.5 $A_{260}$ units of PBN per milliliter. One $A_{260}$ unit refers to the absorbance at 260 nanometers of a 1 milliliter (ml) sample of PBN. One unit is equal to approximately 6 milligrams of salt-free PBN. It is believed that the absorbance measurement of PBN is a more reliable method than a determination by weight of the concentration of PBN in a sample due to the quantity of salt present following the enzymatic hydrolysis of polymyxin B sulfate.

The yeast cell containing the desired cloned gene or genomic DNA coding for the desired polypeptide may be incubated with an effective amount of PBN in any convenient fashion in conventional nutrient media well-known to the art. Following a period of incubation and increased secretion of the desired polypeptide from the cell, said polypeptide is recovered from the incubation milieu by conventional methodologies. One skilled in the art is readily capable of cloning the gene encoding the desired polypeptide (including regulatory regions if desired) into an appropriate vector and transforming the host yeast with said vector. Expression of the polypeptide product from the transformants is enhanced by the method of this

invention. Further, as described herein, the method of the present invention is equally applicable to increase the efficiency of secretion of polypeptides coded for by genomic DNA, i.e., that DNA normally present in a yeast cell coding for the expression of a polypeptide such as invertase or acid phosphatase. Thus, the method of the present invention provides a means for overcoming a serious limitation on the use of microorganisms for industrial-scale production of natural products.

The present invention is applicable to any genera of yeast. However, the greatest impact of the methods described herein will be on those yeast commonly used as hosts for the expression of cloned genes such as various species of *Saccharomyces* and mutants thereof, particularly *Saccharomyces cerevisiae* and mutants thereof.

The invention is further illustrated by the following non-limiting examples.

Polymyxin B sulfate is hydrolyzed by purified ficin in a phosphate buffer (pH 7) for 24 hours at 37°C. The reaction mixture is then boiled (to coagulate the enzyme) and the polymyxin B hydrolysate is clarified by filtration. The clarified solution is then acidified to pH 2 with 1 normal HCl and subsequently extracted with n-butanol to dissolve fatty acyl diaminobutyric acid. The residual aqueous solution is adjusted to pH 8 with 1 normal NaOH and is repeatedly extracted with n-butanol to remove any remaining polymyxin B. The pH of the aqueous layer is again adjusted (i.e., pH

7) and the material is passed through an Amberlite® IRA-410 column (OH type). The effluent is adjusted to pH 5, concentrated in vacuo and lyophilized. Because the resulting PBN contains large amounts of NaCl (3-4 M), the salt may be removed by standard techniques such as filtration, for instance, through a Sephadex® G10 column using ammonium carbonate as the eluent.

Polymyxin B nonapeptide induces a modification in yeast membrane function without consequent damage to the cell (i.e., is nontoxic). The use of PBN to modify the structural integrity of the outer cell membrane of yeast without being toxic to the cell allows facilitated export of polypeptides or other macromolecules from the cell thereby increasing the efficiency of secretion and facilitating recovery of the desired polypeptide product of genomic DNA or cloned genes as described herein.

Enhanced expression of polypeptides such as proinsulin, insulin A or B chain, human or bovine growth hormone, interferon, somatostatin, thymosin, invertase acid phosphatase and the like may be achieved by the method of the present invention. For example, the gene coding for somatostatin is inserted into a suitable cloning vehicle and transformed into *Saccharomyces cerevisiae*. The transformants are incubated with an effective amount of PBN thereby leading to increased levels of secretion of somatostatin relative to transformants not incubated with PBN. The enhanced secretion from those transformants contacted with

PBN is believed to be due to the modification of the integrity of the yeast cell membrane and subsequent facilitated passage of somatostatin out of the cell. The somatostatin may then be recovered by conventional techniques. Additionally, inasmuch as the PBN is nontoxic to the cell, the viability of the transformed cells is not compromised by the presence of PBN in the culture medium hence allowing the cells to produce and secrete additional quantities of somatostatin.

Similarly, a strain of *Saccharomyces cerevisiae* containing genomic DNA for the production and expression of invertase is incubated with an effective amount of PBN. The secretion of invertase from those cells incubated with PBN is increased relative to those cells not incubated with PBN.

What Is Claimed Is:

1. A method of increasing the permeability of yeast cells which comprises incubating said yeast cells with an effective amount of polymyxin B nonapeptide.

2. The method of claim 1 wherein the yeast cell belongs to the genus *Saccharomyces*.

3. The method of claim 2 wherein the yeast is a strain of *Saccharomyces cerevisiae* and mutants thereof.

4. A method for increasing the efficiency of secretion from yeast of polypeptides encoded by genomic DNA or cloned genes which comprises incubating said yeast containing said genomic DNA or cloned genes in the presence of an effective amount of polymyxin B nonapeptide and recovering therefrom said polypeptide.

5. The method of claim 4 wherein the yeast belongs to the genus *Saccharomyces*.

6. The method of claim 5 wherein the yeast is a strain of *Saccharomyces cerevisiae* and mutants thereof.

7. A yeast cell having increased permeability by the method of claim 1.

8. The yeast cell of claim 7 wherein said yeast is a member of the genus *Saccharomyces*

9. The yeast cell of claim 8 wherein said yeast is a strain of *Saccharomyces cerevisiae* and mutants thereof.